Europäisches Patentamt

European Patent Office

Office européen des brevets

(19)

(11) Publication number: **0 213 628**
**A2**

(12) # EUROPEAN PATENT APPLICATION

(21) Application number: 86112075.6

(22) Date of filing: 01.09.86

(51) Int. Cl.⁴: **C 12 N 15/00**
**C 12 N 9/02, C 12 N 5/00**

(30) Priority: 03.09.85 US 772122

(43) Date of publication of application:
**11.03.87 Bulletin 87/11**

(84) Designated Contracting States:
**AT BE CH DE FR GB IT LI LU NL SE**

(71) Applicant: **YEDA RESEARCH AND DEVELOPMENT COMPANY, LTD.**
**Weizmann Institute of Science Herzl Street at Yavne Road**
**P.O. Box 95**
**Rehovot 76100(IL)**

(72) Inventor: **Groner, Yoram Meonot Wolfson A.**
**The Weizmann Institute of Science**
**Rehovot 76000(IL)**

(74) Representative: **Henkel, Feiler, Hänzel & Partner**
**Möhlstrasse 37**
**D-8000 München 80(DE)**

(54) **Expression of superoxide dismutase in eukaryotic cells.**

(57) The invention concerns the production of superoxide dismutase (SOD) by eucaryotic cells derived from multicellular organisms which are capable of expressing a DNA sequence encoding for superoxide dismutase and producing the superoxide dismutase or analogs thereof.

A specific embodiment of the invention includes a human HeLa cell or mouse L cell containing either the pmSV2-SOD-Neo plasmid or the pHG-SOD-SV-Neo plasmid. The pmSV2-SOD-Neo plasmid contains a DNA sequence encoding for superoxide dismutase joined to the SV40 early promoter. The pHG-SOD-SV-Neo plasmid contains a DNA sequence encoding for superoxide dismutase controlled by the native regulatory elements of human genomic Cu-Zn SOD. Such cells are capable of expressing the sequence encoding the superoxide dismutase and of producing the superoxide dismutase or an analog thereof.

The invention also concerns methods of producing the superoxide dismutase by growing the cells of the invention and recovering the superoxide dismutase product.

*1*

# EXPRESSION OF SUPEROXIDE DISMUTASE
## IN EUKARYOTIC CELLS

## Background of the Invention

Throughout this application, various publications are referenced by numbers within parentheses. Full citations for these publications may be found at the end of the specification immediately preceding the claims. The disclosure of these publications in their entireties are hereby incorporated by reference into this application in order to more fully describe the state of the art to which this invention pertains.

Superoxide dismutase (superoxide:superoxide oxidoreductase, EC 1.15.1.1) is the enzyme that catalyzes the removal of superoxide radicals, which are generated in a variety of biological oxidations (23). It provides a defense against oxygen toxicity and damage that may be caused to cells by carcinogenic hydrocarbons (23). The human Cu-Zn superoxide dismutase (SOD-1) is a dimeric protein composed of apparently identical noncovalently linked subunits, each with a molecular weight of 16,000-19,000 (24,25). The locus for human cytoplasmic superoxide dismutase (SOD-1) was assigned to chromosome 21 (26).

About 1 in 600 newborn babies carries an extra chromosome 21, a condition technically known as trisomy 21 or Down Syndrome (27,28). This chromosome imbalance is a known cause of spontaneous abortion and mental retardation (27). In most cases, the patients with Down Syndrome have karyotypes with 47 chromosomes (46 plus one additional 21). However, cases of Down Syndrome in which only a portion of chromosome 21 is present in triplicate have enabled the localization of the "responsible" region to segment 21q22, the distal portion of the long arm (29-33). Although trisomy 21 was identified as a human genetic disease over 20 years ago (27), little is known about the mechanisms by which the extra chromosome or the extra chromosomal segment 21q22 results in reduced viability and abnormalities of morphogenesis and mental function. It is generally assumed that the extra chromosome or chromosomal segment codes for normal products and that the abnormalities found in Down Syndrome are produced by an imbalance due to changes in gene dosage (34). Namely, the presence of additional genetic material in the cell will result in the production of commensurately increased amounts of the gene products coded by the extra chromosomal segment. Indeed, Down Syndrome patients show an increase of about 50% in SOD-1 activity (35-37) due to a higher level of SOD-1 protein (38). However, it is not known whether this gene dosage phenomenon is a result of quantitative changes in the amount of SOD-1 mRNA.

In the past, most of the reports on Down Syndrome involved family karyotyping and clinical studies of the effects of the disease on patients. It is only recently that recombinant DNA techniques have enabled one to appproach the molecular biology of the chromosomal region involved and try to gain insight into the mechanism by which abnormal karyotypes result in abnormal phenotype.

Superoxide dismutase is also of considerable interest because of its pharmacological properties. Bovine-derived, naturally-occurring superoxide dismutase (or-

gotein) has been recognized to possess anti-inflammatory properties and is currently marketed in certain European countries, e.g., West Germany, for use in the treatment of inflammation. It is also sold in a number of countries including the United States as a veterinary product for treating inflammation, particularly for treating inflamed tendons in horses.

Additionally, the scientific literature suggests that SOD may be useful in a wide range of clinical applications. These include prevention of oncogenesis and tumor promotion and reduction of cytotoxic and cardiotoxic effects of anti-cancer drugs (Oberley, L.W. and Buettner, G.R., Cancer Research 39, 1141-1149 (1979)); protection of ischemic tissues (McCord, J.M. and Roy, R.S., Can. J. Physiol. Pharma. 60, 1346-1352 (1982)), and protection of spermatozoa (Alvarez, J.G. and Storey, B.T., Biol. Reprod. 28, 1129-1136 (1983)). In addition, there is a great interest in studying the effect of SOD on the aging process (Talmasoff, J.M., Ono, T. and Cutler, R.G., Proc. Natl. Acad. Sci. USA 77, 2777-2782 (1980)).

The present invention is directed to the expression of DNA encoding superoxide dismutase in eucaryotic cells derived from multicellular organisms, the production of superoxide dismutase by such eucaryotic cells, and superoxide dismutase or analogs thereof so produced.

The gene encoding human cytoplasmic superoxide dismutase has been cloned and a cDNA prepared. See Lieman-Hurwitz, et al., Proc. Natl. Acad. Sci. USA 79, 2808-2811 (1982). The complete sequence of the cloned DNA also has been determined. See Proc. Natl. Acad.

Sci. USA 80, 5465-5469 (1983). See also pending coassigned U.S. application Serial No. 489,786 filed April 29, 1983.

Moreover, the expression of DNA encoding superoxide dismutase in bacterial hosts and the production and recovery of superoxide dismutase produced by such bacteria has also been achieved. See European Patent Publication No. 0131843 Al, published January 23, 1985, corresponding to European Patent Application No. 84107717.5 filed July 3, 1984, which claims priority of U.S. Serial No. 514,188 filed July 15, 1983.

European Patent Publication 0138111 Al of European Patent Application No. 84111416.8 filed September 25, 1984, and claiming priority of U.S. Serial No. 538,607 filed October 3, 1983, and U.S. Serial No. 609,412 filed May 11, 1984, discloses the expression of a superoxide oxide dismutase gene and the production of SOD in yeast, a eucaryotic unicellular microorganism. See also EMBO Journal, Vol. 4, No. 1, pp. 77-84 (January 1985) which is directed to characterizing the SOD-1 gene locus on human chromosome 21.

However, there is no disclosure in the prior art concerning the expression of DNA sequence encoding superoxide dismutase in genetically engineered eucaryotic cells of multicellular organisms, and production of SOD by such cells.

The herbicide paraquat (1,1'-dimethyl-4,4'-bipyridinium dichloride) is known to increase the production of superoxide radicals ($O_2$). It was reported that para-

quat stimulates SOD activity in E. coli (8) and caused lipid peroxidation in mouse lung microsomes (9). The present invention also is directed to SOD DNA expressing cells which are paraquat resistant cells.

Under certain conditions, elevated expression of SOD might be toxic to the cells producing it. During oxidative metabolism, unstable oxygen radicals are formed both spontaneously and as a result of enzymatic activity of oxidative enzymes (17 and 18). Radicals are characterized by a single electron in the outer shell, explaining their high reactivity (18). Cu/Zn-Superoxide dismutase (SOD-1) is a protective enzyme responsible for maintaining lower levels of superoxide radicals within the cell, by catalyzing the interaction of two free radicals in such a way that one is reduced and the other oxidized, the dismutation reaction, resulting in formation of $H_2O_2$ (19).

The superoxide radical ($O_2$) is not particularly toxic. The danger stems from its ability to interact with $H_2O_2$ to generate singlet oxygen ($^1O_2$) and hydroxy radicals (HO·); both are extremely active and highly cytotoxic forms of oxygen (18).

Increased SOD-1 activity could lead to an increased production of $H_2O_2$. The accumulation of peroxides in the cell might exert toxic effects by increased formation of singlet oxygen ($^1O_2$) and hydroxy radicals (HO·) which can react directly with polyunsaturated fatty acids to form hydroperoxides. Also, the arachidonic acid cascade, leading to the biosynthesis of prostaglandins and involving cooxidation of unsaturated fatty acids, is modulated by the intracellular oxidative environment. Therefore, higher SOD-1 activity may cause an increase in lipoperoxidation and a decrease in unsaturated fatty acid content of cell membranes which may cause abnormalities of membrane functions (18 and 20).

Therefore, to obtain a cell line which could produce high levels of SOD-1 but which does not have this toxicity problem would be highly advantageous.

The present invention is also directed to cells in which the expression of DNA encoding superoxide dismutase and production of the superoxide dismutase can be regulated so that high levels of SOD are produced without causing damage to the cells.

Recently, inhibition of thymidine kinase gene expression in mouse L-cells by microinjection of antisense RNA was reported (12). In prokaryotes, translational inhibition by complementary RNA transcript was shown to regulate the expression of the ompF gene in E. coli (12) and an anti-mRNA produced by a plasmid dramatically inhibited (98%) the synthesis of β-galactosides (14).

A particular aspect of the present invention concerns cells in which controlled expression of the SOD gene and production of SOD is achieved by anti-sense SOD mRNA.

## Summary of the Invention

A eucaryotic cell of a multicellular organism in which a DNA sequence encoding superoxide dismutase (SOD) is expressed has been invented. In a preferred embodiment of the invention, this cell has been transformed with a plasmid comprising a DNA sequence encoding superoxide. The sequence encoding for superoxide dismutase can be present in a cell in multiple copies.

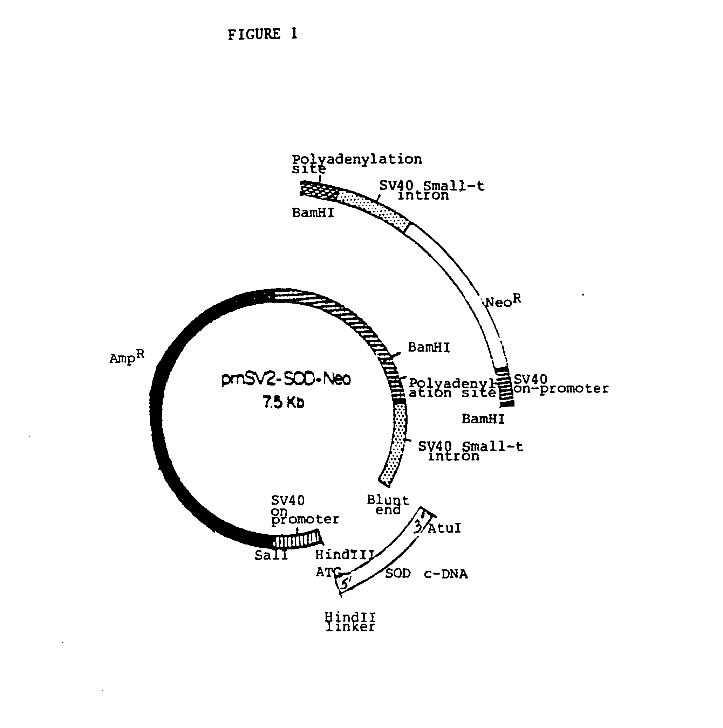

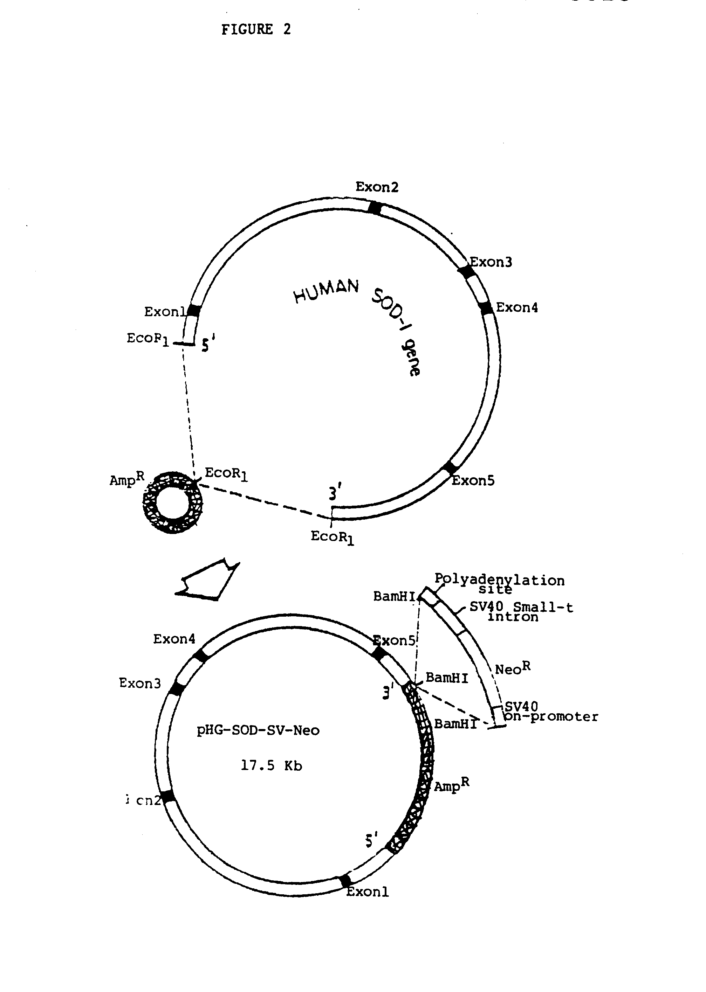

Preferably, the DNA sequence encoding superoxide dismutase encodes human Cu-Zn superoxide dismutase (SOD-1). Examples of DNA sequences encoding Cu-Zn human superoxide dismutase include the cDNA of Cu-Zn human superoxide dismutase and genomic DNA containing the Cu-Zn human superoxide dismutase gene. More preferably, the eucaryotic cell of this invention is transformed with a plasmid which contains a cDNA sequence encoding for human Cu-Zn superoxide dismutase joined to the SV40 early promoter such as the pmSV2-SOD-Neo plasmid, or a plasmid which contains human genomic DNA encoding for human Cu-Zn superoxide dismutase and regulated by the native regulatory elements of that gene, such as the plasmid pHG-SOD-SV-Neo. The plasmid pmSV2-SOD-Neo has the restriction map shown in FIG. 1 and the plasmid pHG-SOD-SV-Neo has the restriction map shown in FIG. 2. Such cells are capable of expressing the DNA sequence encoding the superoxide dismutase and producing superoxide dismutase or an analog thereof.

In a preferred embodiment of the invention, the cell is derived from a mammal and is preferably a human HeLa cell or a mouse L cell. In another preferred embodiment of the invention, the cell is derived from a plant.

The invention also concerns clones of the cells produced in accordance with the methods of the invention and cell lines produced from these cells.

Another aspect of this invention is the production of superoxide dismutase or an analog thereof by growing the cells of this invention in a suitable medium under suitable conditions permitting production of the superoxide dismutase or analog thereof and recovering the resulting superoxide dismutase or analog thereof. The invention also concerns the superoxide dismutase or analog thereof produced by the methods of this invention, preferably those having the activity of naturally occurring superoxide dismutase, as well as compositions containing the same.

The invention is also directed to a cell, especially a eucaryotic cell, in which a DNA sequence encoding superoxide dismutase is expressed and which is resistant to paraquat.

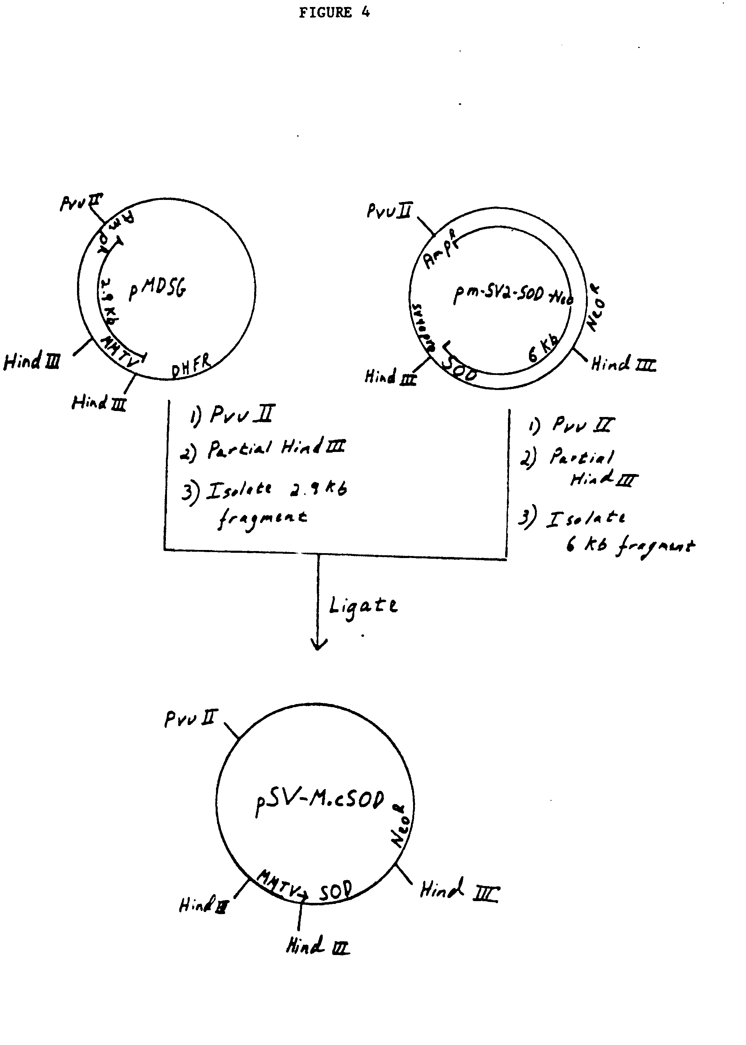

Eucaryotic cells in which a DNA sequence encoding superoxide is programably expressed and in which the production of SOD can be regulated are also the subject of this invention. In a specific embodiment of the invention, a eucaryotic cell is transformed with a plasmid containing a DNA sequence encoding superoxide dismutase joined to an inducible promoter. Preferably, the inducible promoter is the mouse mammary tumor virus (MMTV) promoter and more preferably, the cell is transformed with the plasmid pSV-M.cSOD having the restriction map shown in FIG. 4.

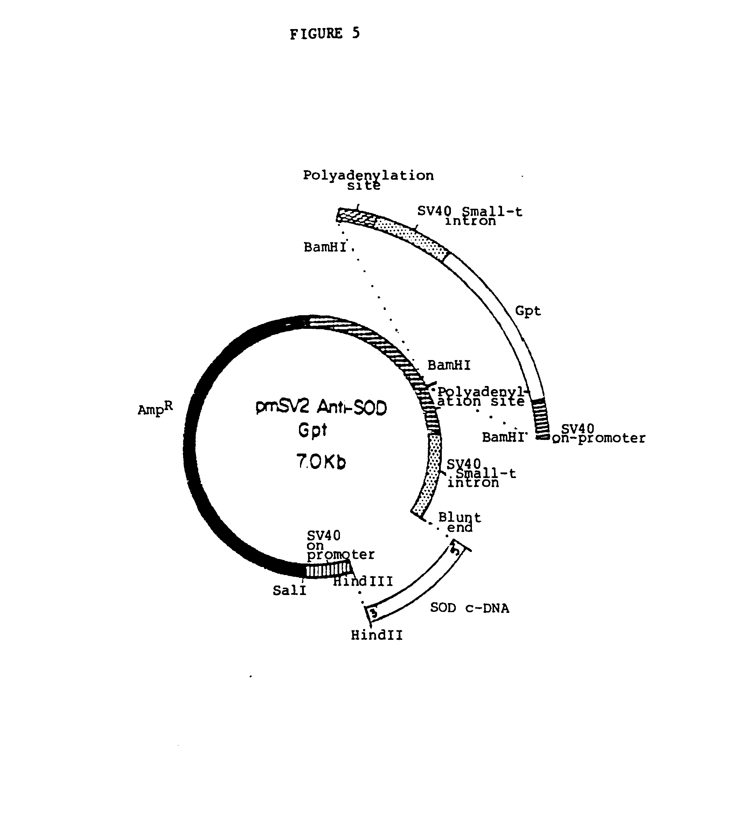

The invention also includes cells in which a DNA sequence encoding anti-sense superoxide dismutase mRNA is expressed. Preferably the cell is a eucaryotic cell and the DNA sequence encodes anti-sense human Cu-Zn superoxide dismutase. More preferably the cell has been transformed with the plasmid pmSV-anti-SOD-gpt having the restriction map shown in FIG. 5. The invention also includes cells, especially eucaryotic cells, in which a DNA sequence encoding superoxide dismutase and a DNA sequence encoding anti-sense superoxide dismutase mRNA are both expressed.

## Description of the Figures

FIG. 1: Structure of plasmid pmSV2-SOD-Neo, a SOD-1 cDNA transducing vector.

pBR322 DNA is represented by the solid black segment. The longer open arc represents the neo gene which directs expression of an aminoglycoside phosphotransferase and the shorter open arc represents the SOD-1 cDNA. The hatched segment contains the SV40 origin of DNA replication and early promoter.

FIG. 2: Structure of plasmid pHG-SOD-SV-Neo, a SOD-1 genomic DNA transducing vector.

pBR322 DNA is represented by black cross-hatched segments. The solid black regions are the exons of the genomic SOD-1 gene. The open arc represents the neo gene which directs expression of an aminoglycoside phosphotransferase. The SOD-1 expression in this vector is regulated by the native regulatory elements of the genomic SOD-1 gene.

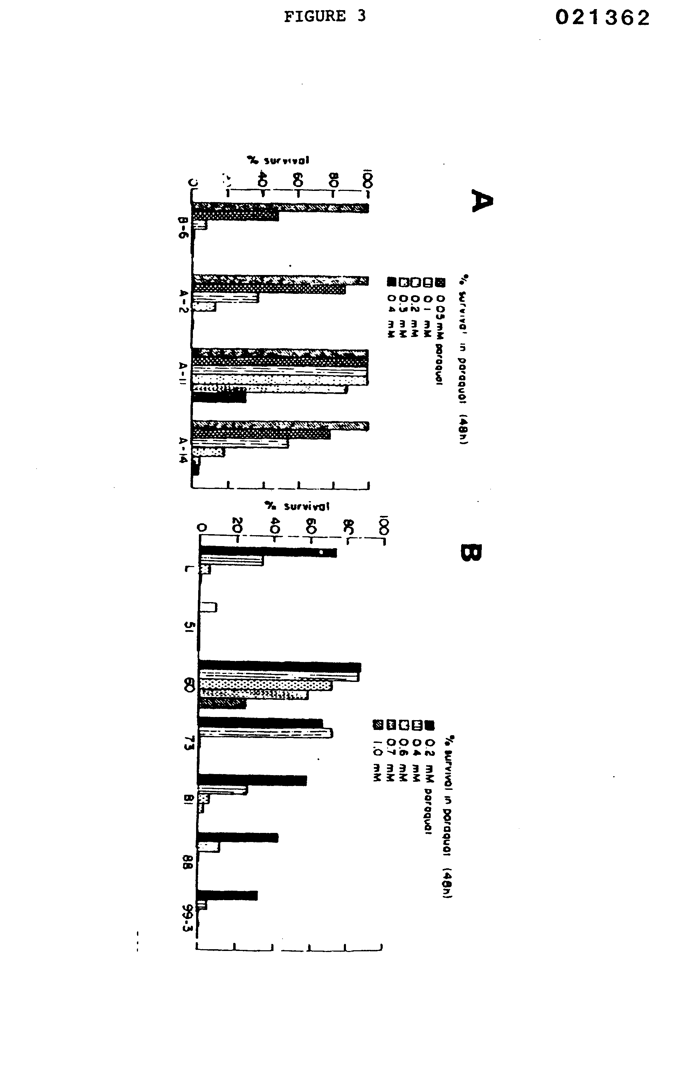

FIG. 3: Graphs demonstrating how the expression of SOD-1 affects paraquat-mediated cytotoxicity.

SOD-1 transformed sub-lines were grown in Dulbecco's modified Eagle's medium supplemented with 10% fetal calf serum. Twenty-four hours before application of paraquat, cells were seeded in 9 mm microwells at 4 x $10^4$ cells/100 µl/well. Paraquat was applied in triplicate at the concentrations indicated and cells were further incubated for 48 hours. Viability of the cells was determined by incubation with neutral red for 2 hours, washing away excess dye, extracting the neutral red that was taken up by the cells with Sorenson's citrate buffer-ethanol mixture and quantitating it colorimetrically at 570 nm with a MicroELISA Autoreader (Dynatech. Alexandria, VA).

Chart A shows HeLa cell derived SOD-1 transformants: B-6 is G418 resistant, SOD-1 negative; A-2, A-11, A-14 are sub-lines which overexpress various levels of recombinant SOD-1.

Chart B shows mouse L cell derived SOD-1 transformants: 51 is G418 resistant, human SOD-1 negative; 60, 73, 81, 88, 99-3 are sub-lines which overexpress various levels of human SOD-1.

FIG. 4: Construction of plasmid pSV-M.cSOD.

The plasmid pMDSG was digested with PvuII and then partially digested with HindIII. A 2.9 Kb fragment containing the mouse mammary tumor virus (MMTV) promoter was isolated. The plasmid pm-SV2-SOD-Neo shown in FIG. 1 was separately digested with PvuII, and then partially digested with HindIII. A 6 Kb fragment containing the SOD-1 and the Neo genes was isolated and ligated to the 2.9 Kb fragment from pMDSG to form the plasmid pSV-M.cSOD which contains the SOD-1 gene regulated by the MMTV inducible promoter.

FIG. 5: Structure of plasmid pmSV2 Anti-SOD Gpt, a plasmid vector for production of "anti-sense" SOD-1 mRNA.

The construct is similar to the one depicted in FIG. 1 except here the SOD-1 cDNA is inverted 3' to 5' and the bacterial gene is Gpt.

## Detailed Description of the Invention

A eucaryotic cell of a multicellular organism in which a DNA sequence encoding superoxide dismutase is expressed has been invented. A eucaryotic cell is transformed by methods known to those of ordinary skill in the art with a plasmid comprising a DNA sequence encoding for superoxide dismutase. The transformed cells in which the sequence encoding for the superoxide dismutase is present in multiple copies can be selected. These transformed eucaryotic cells capable of expressing a DNA sequence encoding for superoxide dismutase are also capable of producing superoxide dismutase or analogs thereof.

The DNA sequence encoding for superoxide dismutase may be, among others, a cDNA clone which contains the entire coding region for Cu-Zn human superoxide dismutase or a fragment of human genomic DNA containing a Cu-Zn human superoxide dismutase gene isolated from the human genomic library.

In specific embodiments of the invention, the eucaryotic cell is transformed with a plasmid containing a cDNA clone encoding Cu-Zn superoxide dismutase joined to the SV40 promoter. The plasmid pmSV2-SOD-Neo having the restriction map shown in FIG. 1, is an example of such a plasmid.

In other specific embodiments of the invention, the eucaryotic cell is transformed with the plasmid containing a fragment of human genomic DNA containing the Cu-Zn human superoxide dismutase gene controlled by the regulatory elements of the native human Cu-Zn superoxide dismutase gene. Plasmid pHG-SOD-SV-Neo having the restriction map shown in FIG. 2, is an example of such a plasmid.

The eucaryotic cells of this invention may be any eucaryotic cell derived from a multicellular organism. The cell may be derived from a mammal or a plant. Preferred mammalian cells include the human HeLa cell and the mouse L cell.

The cells of this invention may also be transformed with a DNA sequence for a selectable marker, which DNA sequence is preferably on the same plasmid or vector as the DNA sequence encoding superoxide dismutase. Antibiotic resistance is a preferred selectable marker, especially resistance to neomycin and/or aminoglycoside G-418.

A cell in which a DNA sequence encoding superoxide dismutase is expressed at an activity level effective for resistance to the herbicide paraquat is preferred.

The invention also concerns clones of the transformed eucaryotic cell and cell lines derived from these cells.

The invention also concerns superoxide dismutase or analogs thereof, especially those having the activity of naturally-occurring superoxide dismutase, produced by the cells and methods of this invention as well as compositions containing the same.

The method for producing superoxide dismutase of this invention comprises growing the cells in a suitable medium under suitable conditions permitting production of the superoxide dismutase or an analog thereof and recovering the resulting superoxide dismutase or analog thereof.

The invention is also directed to cells in which a DNA sequence encoding superoxide dismutase is programably expressed, i.e., cells wherein the expression of the DNA sequence encoding superoxide dismutase can be regulated. A specific embodiment of this. invention are eucaryotic cells which have been transformed with a plasmid containing a DNA sequence encoding superoxide dismutase joined to an inducible promoter. Preferably, the inducible promoter is mouse mammary tumor virus (MMTV) promoter and more preferably the plasmid is pSV-M.cSOD having the restriction map shown in FIG. 4.

Another embodiment of this invention is a cell in which a DNA sequence encoding anti-sense superoxide dismutase mRNA is expressed, e.g., a cell which has been transformed with a plasmid encoding anti-sense superoxide dismutase mRNA. The plasmid pmSV2-anti-SOD-Gpt having the restriction map shown in FIG. 5 is an example of such a plasmid. Moreover, a cell, preferably a eucaryotic cell, in which both a DNA sequence encoding superoxide dismutase and a DNA sequence encoding anti-sense superoxide dismutase mRNA are expressed is preferred.

## Experimental Procedures

Cell culture and selection of transformants: Human and mouse L cells were maintained in Dulbecco's modified Eagle's medium containing pencillin, streptomycin and 5% newborn calf serum. The antibiotic G418 (Gibco, USA) was stored in Dulbecco's modified Eagle's medium (4 mg/ml) and diluted into culture medium as needed.

Supercoiled plasmid DNA was introduced into tissue culture cells (10 µg for about $5 \times 10^6$ cells) as a calcium phosphate precipitate (21, 22) followed by a glycerol shock after 4 hours. About 48 hours later, the cells were trypsinized and replaced at 1:10 dilution. Selection for $G^{418}$ at 400 µg/ml was instituted 16 hours later.

DNA-blot hybridization: DNA was extracted from G418-resistant human HeLa cells. 10-20 µg of DNA were digested with the appropriate restriction endonuclease and fractionated on a 0.8% agarose gel in TAE buffer (40 mM Tris, 20 mM Na acetate, 2 mM EDTA), containing 1 µg/ml ethidium bromide. The DNA was denatured and transferred to nitrocellulose. Hybridizations with $^{32}$p-labelled pmSV2-Neo DNA were carried out at 42°C in 50% formamide, 5 x SSC, 5 x Denhardt, 50 mM $NaPO_4$ (pH 6.5) and 100 µg/ml of salmon sperm DNA. The filters were washed at 50°C with 0.1 x SSC 0.1% SDS.

Labeling, extraction and immunoprecipitation of SOD-1 from human and mouse cells: Cultures of $8 \times 10^6$ cells were labeled for 6-7 hours with 100 c/ml of $[^3H]$-leucine (50 Ci/mmole) in leucine-free medium. Plates were washed twice with cold phosphate buffered solution (PBS) and cells were lysed with 1.8 ml/plate of freshly prepared extraction buffer containing 20 mM NaCl, 1 mM MgAc, 1 mM $CaCl_2$, 0.5% NP-40, 0.1 mg/ml 2-1-tosylamide-2-phenyl-ethylchloromethyl ketone and 0.2 mg/ml phenylmethyl-sulfonyl-fluoride. Nuclei were removed by centrifugation at 10,000 xg for 2 minutes and cytoplasmic extract saved for immunoprecipitation. To 200 µl aliquots of extract, 20 µl of rabbit anti SOD-1 were added and the mixture was made 0.1% with respect to sodium dodecyl sulfate (SDS). Incubation was carried out for

1 hour at 23°C, followed by the addition of 100 µl of a 10% suspension of heat-inactivated, formaldehyde fixed Staphylococcus cultures and incubation continued at 23°C for 20 minutes. Bacterial pellets containing the adhered immunoglobulin complexes were precipitated by centrifugation at 10,000 xg for 5 minutes and washed four times in 0.5 to 0.7 ml of buffer containing 20 mM Tris-HCl (pH 7.5), 140 mM NaCl, 10 mM EDTA 0.5% NP-40 and 0.1% SDS. After the final washing the pellets were suspended in 50 µl of electrophoresis sample buffer (6.2 mM) Tris-HCl (pH 6.8), 3% SDS, 5% $\beta$-mercaptoethanol, 10% glycerol) and heated at 100°C for 5 minutes. Samples were centrifuged at 10,000 xg for 5 minutes and 20 µl of the supernatant analyzed by SDS-polyacylamide gel electrophoresis. Alternatively, the polypeptide could be labelled with [35S] cysteine and the same procedures followed.

Polyacrylamide gel electrophoresis: Labeled immunoprecipitates in electrophoresis sample buffer were heated at 100°C for 5 minutes prior to electrophoresis at 150 V for 2.5 hours in 15% slab SDS polyacrylamide gels. Gels were fixed and stained with Coomassie brilliant blue, subjected to fluorography and autoradiographed at -80°C using preexposed Agfa Curix RP-2 film.

Gel electrophoresis and enzymatic assay of superoxide dismutase: An extract (NP-40) of 0.5-1.0x10^6 cells was made, loaded on 10% acrylamide/urea gel and electrophoresis was performed for 3 hours at 150V. Gels and running buffer were without SDS and samples were not heated prior to electrophoresis. Superoxide dismutase was localized by soaking the gels in 2.45 x 10^{-3}M nitroblue tetrazolium for 20 minutes, followed by immer-

sion for 15 minutes in a solution containing 0.028 tetramethyl-ethylenediamine, 2.8 x $10^{-5}$M riboflavin and 0.036M photasium phosphate at pH 7.8. The gels were then placed in glass trays and illuminated for 5 to 15 minutes. During illumination the gels became uniformly blue except at positions containing superoxide dismutase (16). Illumination was discontinued when maximum contrast between the achromatic zone and the gener.' blue color had been achieved. The gels were then photographed.

0213628

-19-

## EXAMPLES

The examples which follow are set forth to aid in understanding the invention but are not intended to, and should not be construed to, limit its scope in any way. The examples do not include detailed descriptions for conventional methods employed in the construction of vectors, the insertion of genes encoding polypeptides of interest into such vectors or the introduction of the resulting plasmids into hosts. Such methods are well-known to those of ordinary skill in the art and are described in numberous publications including by way of example the following:

T. Maniatis, E.F. Fritsch and J. Sambrook, Molecular Cloning: A Laboratory Manual, Cold Spring Harbor Laboratory, New York (1982).

Methods in Enzymology, Vol. 65, "Nucleic Acids (Part I)," edited by Lawrence Grossman and Kivie Moldave, Academic Press, New York (1980).

Methods in Enzymology, Vol. 68, "Recombinant DNA," edited by Ray Wu, Academic Press, New York (1981).

Methods in Enzymology, Vol. 100, "Recombinant DNA (Part B)," edited by Ray Wu, Lawrence Grossman and Kivie Moldave, Academic Press, New York (1983).

Methods in Enzymology, Vol. 101, "Recombinant DNA (Part C)," edited by Ray Wu, Lawrence Grossman and Kivie Moldave, Academic Press, New York (1983).

Principles of Gene Manipulation, An Introduction to Genetic Engineering, 2nd Edition, edited by R.W. Old

-20-

and S.B. Primrose, University of California Press (1981).

Example 1

Construction of Plasmids for Expression of SOD-1 in Eukaryotic Cells

One of the cDNA clones which contains the entire coding region for SOD-1 (1) was inserted in the proper orientation into pmSV2 vector (2, 3) between the SV40 promoter and small-t introns (FIG. 1). A 2.7 Kb BamHI fragment containing the neo-transcriptional unit was then inserted into the BamHI site 3'-proximal to the SOD-1 cDNA. The neo unit directs the expression of an aminoglycoside phosphotransferase which confers upon cells resistance to the toxic aminoglycoside G-418 (3). In this construct both the neo and SOD-1 genes are under the control of SV40 regulatory elements.

A similar vector containing the cellular SOD-1 gene isolated from the human genomic library and regulated by the native regulatory elements of that gene (4) was also constructed (FIG. 2).

Example 2

Production of SOD-1 in Genetically Engineered Eucaryotic Cells

Both human HeLa cells and mouse L cells were used as hosts for the SOD-1 recombinant plasmids. Cells were transfected by the calcium phosphate precipitation method (5) and selected initially for G-418 resistance. Only those cells that express the bacterial phosphotransferase gene (neo) survived.

A number of cell lines that express the vector-derived human SOD-1 were isolated from the G-418 resistant transformants. Among the HeLa derived sub-lines the A-2, A-14 and A-11 contain about two copies of the plasmid integrated in their genome. When assayed for SOD-1 activity, it was found that they express 2-3 fold more SOD-1 activity. Other HeLa-derived sub-lines B-6, B-11 and B-19 were all resistant to G-418 but did not express the vector derived SOD-1.

The mouse L cell derivatives expressing the human SOD-1 gene were analyzed by both immunoprecipitation and enzymatic activity. Positive sub-lines, like G-60, produce elevated levels of human SOD-1 while the negative ones, like G-51, did not express human SOD-1.

Example 3

Effects of Paraquat on Cells Genetically Engineered to Express SOD

Cells were treated for 48 hours with different concentrations of paraquat (1,1'-dimethyl-4,4'-bipyridinium dichloride) and the extent of survival was determined (FIG. 3A). The HeLa derived high SOD-1 sub-line A-11 was totally resistant to paraquat concentrations which killed all the cells of the B-6 sub-line (B-6 are G-418 resistant but express only the native SOD-1). The A-2 sub-line which overexpressed SOD-1 to a lesser extent than A-11 was less resistant to paraquat but not as sensitive as B-6. Surprisingly, A-14 which contained higher levels of SOD-1 activity than A-11, was less resistant (FIG. 3A). Similar results were obtained with mouse L-cell sub-lines (FIG. 3B). G-51, which is G-148 resistant but human SOD-1 negative, was sensitive to 0.2 and 0.4 mM of paraquat, whereas G-88, a high SOD-1 producer, was not affected by these concentrations. Again, G-99/C which possessed higher SOD-1 activity than G-88 was less resistant to the drug.

Example 4

Construction of a Plasmid pSV-M.cSOD Containing SOD-1 cDNA Under the Transcriptional Control of the MMTV Inducible Promoter

Under certain conditions, elevated expression of SOD-1 might be toxic to the cells producing it. To overcome the toxicity problem yet at the same time obtaining a cell line which could produce high levels of SOD-1, we placed the SOD-1 cDNA under the regulation of the mouse mammary tumor virus (MMTV) promoter, whose activity could be controlled by glucocorticoid hormones (10, 11). The plasmid pMDSG containing the dexamethasome-inducible promoter carried on the long terminal repeat of the MMTV genome was provided by Gordon Ringold (FIG. 4). It was cut with PvuII and HindIII and ligated to the SOD-1 cDNA of the pSV2-SOD-Neo vector (FIG. 4) to form plasmid pSV-M.cSOD (FIG. 4).

## Example 5

### Construction of Plasmid pmSV2 Anti-SOD Gpt, an "Antisense SOD-1-mRNA" Producing Vector

The vector illustrated in FIG. 5 has been constructed. It contains the SOD-1 cDNA in an inverted (3' $\longrightarrow$ 5') orientation, and as the selectable marker the bacterial gene xanthine-guanine phosphoribosyl transferase (gpt). Expression of this E. coli enzyme confers upon recipient mammalian cells the capability to utilize xanthine as a purine salvage substrate in mycophenolic acid-xanthine-HAT selective medium (15). The gpt selectable marker permits introduction of this plasmid into the high SOD-1 producing G-418 resistant cells, e.g. A-14 and G-88.

## REFERENCES

1. Sherman, L., Dafni, N., Lieman-Hurwitz, J. and Groner, Y., *Proc. Natl. Acad. Sci. USA* 80: 5465-5469 (1983).

2. 'Mulligan, R.C. and Berg, P., *Science* 209:1422-1427 (1980).

3. Southern, P.J. and Berg, P., *J. Molec. App. Genet.* 1:327-341 (1982).

4. Levanon, D., Lieman-Hurwitz, J., Dafni, N., Wigderson, M., Sherman, L., Bernstein, Y., Laver-Rudich, Z., Danciger, E., Stein, O. and Groner, Y., *EMBO J.* 4:77-84 (1985).

5. Graham, F.L. and Van der Eb, A.J., *Virology* 52:456-467 (1973).

6. Raziuddin, A., Sarkar, F.H., Dutkowski, R., Shulman, L., Ruddle, F.H. and Gupta, S., *Proc. Natl. Acad. Sci. USA* 81:5504-5508 (1984).

7. Foxall, D. and Cohen, J.S., *J. Mag. Reson.* 52:346-349 (1983).

8. Hassan, H.M. and Fridovich, I., *J. Biol. Chem.* 254:10846-10852 (1979).

9. Bus, J.S., Aust, S.D. and Gibson, J.E., *Res. Commun. Chem. Pharmac.* 11:21-38 (1975).

10. Parks, W.P., Scolnick, E.M. and Kozikowski, E.H., *Science* 184:158-160 (1974).

11. Ringold, G.M., Vamaoto, K.R., Tomkins, G.M., Bishop, J.M. and Varmus, H.E., *Cell* 6:299-305 (1975).

12. Izant, J.G. and Weintraub, H. *Cell* 36:1007-1015 (1984).

13. Mizuno, T., Chou, M.Y. and Inouye, M., *Proc. Natl. Acad. Sci. USA* 81:1966-1970 (1984).

14. Pestka, S., Daughterty, B.L., Jung, V., Hotta, K. and Pestka, R.K., *Proc. Natl. Acad. Sci. USA* 81:7525-7528 (1984).

15. Mulligan, R.C. and Berg, P., *Proc. Natl. Acad. Sci. USA* 78:2072-2076 (1981).

16. Lieman-Hurwitz, J., Wolf, D., Goldman, D. and Groner, Y., *Biochem. Int.* 3:107-115 (1981).

17. Malmstrom, B.G., *Ann. Rev. Biochem.* 51:21-59 (1982).

18. Badwey, J.A. and Karnovsky, M.L., *Ann. Rev. Biochem.* 49:695-726 (1980).

19. Fridovich, I. in Advances in Inorganic Chemistry, Eichorn, G.L. and Marrilli, L.G. Eds. Elsevier/North Holland, New York, pp. 67-90 (1979).

20.   Pryor, W.A. Ed. Free Radicals in Biology, Academic Press, New York (1984).

21.   Graham, F.L. and Van der Eb, A.J., <u>Virology</u> 52:456-467 (1973).

22.   Wigler, M., Pellicer, A., Silverstein, S., and Axel, R., <u>Cell</u> 14:725-731 (1978).

23.   Fridovich, I., <u>Annu. Rev. Biochem.</u> 44:147-159 (1975).

24.   Hartz, J.W. and Deutsch, F.H., <u>J. Biol. Chem.</u> 247:7043-7050 (1972).

25.   Lieman-Hurwitz, J., Wolf, D., Goldman, D. and Groner, Y., <u>Biochem. Int.</u> 3:107-115 (1981).

26.   Tan, Y.H., Tischfield, J. and Ruddle, F.H., <u>J. Exp. Med.</u> 137:317-330 (1973).

27.   Lejeune, J.M., Gautier, M. and Turpin, R., <u>C.R. Acad. Sci.</u> 248:1721-22 (1959).

28.   Smith, G.F. and Burg, J.M. in <u>Down's Anomaly</u> (Churchill Livingston, Edinburgh), pp. 234-251 (1976).

29.   <u>Paris Conference 1971:  Standardization in Human Cytogenetics, Birth Defects:  Original Article Series 8</u> (The National Foundation, New York), Vol. 7 (1972).

30.   Niebuhr, E., <u>Humangenetik</u> 21:99-101 (1974).

31. Hagemeijer, A. and Smit, E.M.E., _Hum. Genet._ 38:15-23 (1977).

32. Williams, J.D., Summit, R.L., Martens, P.R. and Kimbrell, R.A., _Am. J. Hun. Genet._ 27:478-485 (1975).

33. Sinet, P.M., Couturier, J., Dutrillaux, B., Poissonnier, M., Rauol, O., Rethore, M., Allard, D., Lejeune, J. and Jerome, H., _Exp. Cell. Res._ 97:47-55 (1976).

34. Kurnit, D.M., Proc. Natl. Acad. Sci. USA 76:2372-2375 (1979).

35. Sichitiu, S., Sinet, P.M., Lejeune, J. and Frezal, J., _Humangenetik_ 23:65-72 (1974).

36. Crosti, N., Sera, A., Rigo, A. and Viglino, P., _Hum. Genet._ 31:197-202 (1976).

37. Feaster, W.W., Kwok, L.W. and Epstein, C.J., _Am. J. Hum. Genet._ 29:563-570 (1977).

38. Frants, R.R., Eriksson, A.W., Jongbloet, P.H. and Hamers, A.J. _Lancet_ ii:42-43 (1975).

WHAT IS CLAIMED IS:

1.  A eucaryotic cell of a multicellular organism in which a DNA sequence encoding superoxide dismutase is expressed.

2.  A cell according to claim 1, wherein the cell has been transformed with a plasmid comprising the DNA sequence encoding superoxide dismutase.

3.  A cell according to claim 2, wherein the sequence encoding superoxide dismutase is present in multiple copies.

4.  A cell according to claim 2, wherein the DNA sequence encoding the superoxide dismutase is the cDNA of Cu-Zn human superoxide dismutase.

5.  A cell according to claim 4, wherein the DNA sequence encoding superoxide dismutase is joined to a SV40 early promoter.

6.  A cell according to claim 5, wherein the plasmid is pmSV2-SOD-Neo having the restriction map shown in FIG. 1.

7.  A cell according to claim 2, wherein the DNA sequence encoding the superoxide dismutase is derived from a fragment of human genomic DNA containing the Cu-Zn human superoxide dismutase gene.

8.  A cell according to claim 7, wherein the DNA sequence encoding superoxide dismutase is regulated by the native regulatory elements of human genomic Cu-Zn superoxide dismutase.

9. A cell according to claim 8, wherein the plasmid is pHG-rSOD-SV-Neo having the restriction map shown in FIG. 2.

10. A cell according to claim 1, wherein the cell is a a mammalian cell.

11. A cell according to claim 10, wherein the cell is a human HeLa cell.

12. A cell according to claim 10, wherein the cell is a mouse L cell.

13. A cell according to claim 1, wherein the cell is a plant cell.

14. A cell according to claim 1, wherein the cell is capable of producing Cu-Zn human superoxide dismutase or an analog thereof.

15. A clone of the cell of claim 1.

16. A cell line obtained from the cell of claim 1.

17. A cell according to claim 1, wherein the cell is resistant to paraquat.

18. A method for producing superoxide dismutase which comprises growing the cell of claim 1 in a suitable medium under suitable conditions permitting production of the superoxide dismutase or an analog thereof and recovering the resulting superoxide dismutase or analog thereof.

19. Superoxide dismutase or an analog thereof produced by the method of claim 18.

20. Cu-Zn human superoxide dismutase or an analog thereof produced by the method of claim 18.

21. Superoxide dismutase or an analog thereof produced by the method of claim 18 having the activity of naturally occurring superoxide dismutase.

22. A composition comprising superoxide dismutase or an analog thereof in accordance with claim 21 and a suitable carrier.

23. A cell according to claim 1, wherein the DNA sequence encoding superoxide dismutase is programably expressed.

24. A cell according to claim 23, wherein the cell has been transformed with a plasmid comprising a DNA sequence encoding superoxide dismutase joined to an inducible promoter.

25. A cell according to claim 24, wherein the inducible promoter is the mouse mammary tumor virus (MMTV) promoter.

26. A cell according to claim 25, wherein the plasmid is pSV-M.cSOD having the restriction map shown in FIG. 4.

27. A cell in which a DNA sequence encoding anti-sense superoxide dismutase mRNA is expressed.

28. A cell according to claim 27, wherein the DNA sequence encodes anti-sense human Cu-Zn superoxide dismutase mRNA.

29. A cell according to claim 28, wherein the cell has been transformed with the plasmid pmSV2-Anti-SOD-gpt, having the restriction map shown in FIG. 5.

30. A cell in which a DNA sequence encoding superoxide dismutase and a DNA sequence encoding anti-sense superoxide dismutase mRNA are expressed.

FIGURE 1

FIGURE 2

FIGURE 3

FIGURE 4

FIGURE 5